# EUROPÄISCHE PATENTSCHRIFT

(11) **EP 3 727 481 B1**
(45) Veröffentlichungstag und Bekanntmachung des Hinweises auf die Patenterteilung: **10.07.2024**
(21) Anmeldenummer: 18825954.3
(22) Anmeldetag: 14.12.2018
(51) Int. Cl.: A61L 15/22, A61L 15/42, A61L 26/00

(54) **PH REGULIERENDE WUNDAUFLAGE**
PH-REGULATING WOUND DRESSING
PANSEMENT RÉGULANT LE PH

(30) Priorität: 21.12.2017 DE 102017130893
(43) Veröffentlichungstag der Anmeldung: 28.10.2020
(73) Patentinhaber: Paul Hartmann AG, 89522 Heidenheim (DE)
(72) Erfinder: KETTEL, Markus, 89520 Heidenheim (DE); JUNGINGER, Martin, 89568 Hermaringen (DE)
(74) Vertreter: Paul Hartmann AG Patents & Licensing
(86) Internationale Anmeldenummer: PCT/EP2018/084904
(87) Internationale Veröffentlichungsnummer: WO 2019/121376

(56) Entgegenhaltungen:
- WO-A1-02/47737
- DE-A1- 102011 106 046
- ELISABETTA ESPOSITO ET AL: "Spray dried Eudragit microparticles as encapsulation devices for vitamin C", INTERNATIONAL JOURNAL OF PHARMACEUTICS, vol. 242, 1 January 2002 (2002-01-01), pages 329 - 334, XP055572997
- MOHAMED A. ALHNAN ET AL: "In-Process Crystallization of Acidic Drugs in Acrylic Microparticle Systems: Influence of Physical Factors and Drug-Polymer Interactions", JOURNAL OF PHARMACEUTICAL SCIENCES, vol. 100, no. 8, 1 August 2011 (2011-08-01), US, pages 3284 - 3293, XP055573001, ISSN: 0022-3549, DOI: 10.1002/jps.22572

## Beschreibung

Die vorliegende Erfindung betrifft Wundauflagen, insbesondere zur bedarfsgerechten Behandlung von chronischen und/oder infizierten Wunden.

Die Heilung von Hautwunden beruht auf der Fähigkeit der Haut, Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben. So werden in der Literatur drei wesentliche Heilungsphasen einer Wunde, insbesondere bei Wunden mit Gewebeverlust, beschrieben. Hierzu gehört die Entzündungs- (inflammatorische) oder exsudative Phase zur Blutstillung und Wundreinigung (Phase 1, inflammatorische Phase oder Reinigungsphase), die proliferative Phase zum Aufbau von Granulationsgewebe (Phase 2, Proliferations- oder Granulationsphase) und die Differenzierungsphase zur Epithelisierung und Narbenbildung (Phase 3, Differnzierungs- oder Epithelisierungsphase). Es hat sich gezeigt, dass eine Abheilung der Wunde durch eine moderne, feuchte Wundbehandlung besonders gefördert wird. Im Rahmen der feuchten Wundbehandlung werden unter anderem Wundauflagen mit einer Schaumschicht eingesetzt. Solch eine Schaumschicht bietet nachwachsendem Gewebe eine Matrix, die die Wundheilung stimuliert, und kann gleichzeitig größere Mengen Wundexsudat aufnehmen und binden. Außerdem hat es sich bewährt, für die Feuchthaltung von Wunden Hydrogele einzusetzen, die zudem Hautirritationen am Wundrand verringern.

Es ist seit Langem bekannt, dass es bei der Wundheilung, insbesondere während der Entzündungs- und der Granulationsphase der Wundheilung, zu Störungen kommen kann. Im Zusammenhang mit der vorliegenden Erfindung schließt der Begriff Wunde den Wundgrund ein. In der Wunde kann sich Wundexsudat befinden. Die Wundheilung kann dabei vom pH-Wert einer Wunde bzw. vom pH-Wert des in der Wunde enthaltenen Wundexsudats beeinflusst werden. Gesunde Haut weist üblicherweise einen pH-Wert im Sauren auf, der ungefähr zwischen pH 4,0 bis pH 5,7 liegt.

Bei Wunden, welche eine gestörte Wundheilung aufweisen, insbesondere bei chronischen oder infizierten Wunden, kann häufig ein alkalischer pH-Wert der Wunde oder des Wundexsudats beobachtet werden. Die Messung kann beispielsweise durch eine in-vitro-Messung des pH-Werts von Wundexsudat erfolgen. Eine Verschiebung des pH-Wertes vom Sauren ins Alkalische kann beispielsweise durch die Vermehrung von Bakterien oder durch die Bildung von Nekrosen verursacht werden.

Nekrosen und pathologische Mikroorganismen können auf den physiologischen Metabolismus während des Wundheilungsgeschehens einwirken. Dies führt häufig zu lokaler Hypoxie, und dann zum weiteren Abbau umliegenden Gewebes. Das entstehende alkalische Milieu kann weitere gewebeabbauende Prozesse begünstigen. Weiterhin kann das alkalische Milieu die Vermehrung weiterer pathogener Mikroorganismen stimulieren und so die Wundheilung zusätzlich behindern. Als chronische Wunden werden im Rahmen dieser Erfindung Wunden bezeichnet, die nicht in einem erwarteten Zeitraum von 4 bis 6 Wochen verheilen.

Die inflammatorische Phase tritt üblicherweise direkt nach dem Trauma auf und dauert in etwa drei Tage. Sie ist gekennzeichnet durch Gefäßkontraktion, Aktivierung der Gerinnungskaskade und komplexe immunologische Abläufe. Es kommt in der Regel zur Ausbildung eines Fibrinnetzes, welches die Wunde verschließt und nach außen schützt. Durch die Freisetzung vasoaktiver Substanzen (z.B. Histamin und Serotonin) kann eine lokale Entzündungsreaktion hervorgerufen werden. Die umliegenden Gefäße können sich erweitern und durch eine gesteigerte Kapillarpermeabilität können Leukozyten zum Entzündungsort wandern. Diese können Mikroorganismen und Gewebsnekrosen beseitigen. Dadurch kann eine Reinigung der Wunde erfolgen.

Die darauffolgende Proliferations- oder Granulationsphase beginnt üblicherweise etwa am zweiten Tag nach der Wundentstehung und kann z.B. bis zu 14 Tage andauern. Es erfolgt der Aufbau von neuem Gewebe mit Gefäßeinsprossung und Defektauffüllung durch Granulationsgewebe. Dies ist die Grundvoraussetzung für die spätere Epithelisierung. Fibroblasten aus dem umliegenden Gewebe können in das Fibrinnetz migrieren und es als provisorische Matrix nutzen. Es beginnt der Aufbau von Kollagenfasern. Durch das Enzym Plasmin kann das Fibringerüst mittels Fibrinolyse abgebaut werden. Die verschlossenen Gefäße können rekanalisiert werden.

Mit der Differenzierungs- oder Umbauphase beginnt, etwa zwischen dem sechsten und zehnten Tag, üblicherweise die Ausreifung der kollagenen Fasern. Die Wunde kontrahiert sich durch die Umwandlung von Fibroblasten in Fibrozyten sowie Myofibroblasten. Dadurch schrumpft das Narbengewebe und es führt zu einer Verkleinerung der Wunde. Die Epithelisierung vom Wundrand her bringt die Wundheilung zum Abschluss.

Chronische Wunden können definiert werden als Wunden, deren Heilungsverlauf in einem oder allen Stadien der Wundheilung von der normalen Wundheilung abweichen. So kann aus akuten, normal heilenden Wunden z.B. durch eine Wundinfektion eine chronische Wunde entstehen, die durch eine verzögerte Heilungsgeschwindigkeit gekennzeichnet ist. Der Übergang von einer akuten zu einer chronischen Wunde kann dabei in jedem Stadium der Wundheilung erfolgen. Klinisch werden chronische Wunden definiert als Wunden, deren Heilung mehr als 6-8 Wochen benötigen, wobei diese Definition nicht alle Krankheitsbilder korrekt abdeckt. Es handelt sich bei chronischen Wunden mehr um eine Diagnose, die sich auf die klinische Erfahrung des medizinischen Personals stützt.

Chronische Wunden entstehen insbesondere aufgrund einer mechanischen Belastung (Dekubitus, Druck-Ulzera, Druckgeschwür), einer venösen Insuffizienz (Ulcus cruris venosum, venöse Ulzera), einer artheriosklerotischen Gefäßveränderung (Ulcus cruris arteriosum, arterielle Ulzera), einer neuropathischen Veränderungen (diabetisches Fußsyndrom, neuropathische Ulzera), aber auch in Folge von Autoimmunerkrankungen, von Tumoren (exulzierende Tumore) oder Strahlenschäden bei der Tumortherapie.

EP901795 offenbart bioabsorbierbare Materialien, die Puffersubstanzen enthalten und so einen pH-Wert von 4,5 bis 6,5 stabilisieren. Diese Materialien lassen sich auch als Wundversorgungsprodukte verwenden, wobei sie auf eine Wunde aufgelegt werden und durch den Kontakt mit Wundexsudat aufgelöst werden. Diese Wundversorgungsprodukte verbleiben auf der Wunde bis zur vollständigen Resorption durch den Körper.

WO02/47737 offenbart eine Wundauflage mit einem absorbierenden Hydrogel und einer durch eine Sperrschicht umschlossene zusätzliche absorbierende Schicht. Die Sperrschicht ist bei einem leicht alkalischen pH-Wert besser löslich als im neutralen Milieu. Anfallendes Wundexsudat wird zunächst von der Hydrogelschicht absorbiert.

WO02/28447 offenbart einen hautfreundlichen Klebstoff, der eine Substanz enthält, die bei Kontakt mit pH-neutralem Wasser den pH-Wert des Wassers absenkt. Das Dokument offenbart auch eine Wundauflage, welche auf der wundzugewandten Seite mit besagtem Klebstoff beschichtet ist.

EP2726113 offenbart ein Wundversorgungsprodukt mit einem schaumförmigen absorbierenden Material, welcher Puffersubstanzen enthält.

WO2014/027017 offenbart die Verwendung von Puffersubstanzen in der Wundbehandlung zur Unterdrückung der Fibrinbildung sowie Wundversorgungsprodukte, die derartige Puffersubstanzen enthalten.

Sämtliche der genannten Dokumente offenbaren Wundauflagen enthaltend Puffersubstanzen zur Senkung des pH-Werts. Allen dieser Offenbarungen ist gemein, dass die Puffersubstanzen unkontrolliert bzw. nicht bedarfsgerecht an die Wund abgegeben werden.

Die im Stand der Technik beschriebenen Nachteile werden durch einen Gegenstand der vorliegenden Erfindung überwunden, wie er in den Ansprüchen definiert ist.

Die vorliegende Erfindung betrifft Wundauflagen enthaltend eine Brönstedsäure und ein pH-sensitives Polymer, worin die Brönstedsäure in eine Matrix aus pH-sensitivem Polymer eingebettet ist oder von pH-sensitivem Polymer umhüllt ist, wobei es sich bei der Brönstedsäure um eine Substanz ausgewählt aus der Gruppe Essigsäure, Zitronensäure, Milchsäure, Glycerinsäure, Gluconsäure, Benzoesäure, Aconitsäure, Glutarsäure, Weinsäure, Phosphorsäure, Äpfelsäure, Bernsteinsäure und Glutaminsäure handelt und wobei es sich bei dem pH-sensitiven Polymer um ein Methacrylsäure-comethylmethacrylat-Polymer handelt.

Im Rahmen der vorliegenden Erfindung wird unter einer Wundauflage ein Gegenstand verstanden, welcher auf einer Wundoberfläche an einem Patienten angebracht wird. Bei einer erfindungsgemäßen Wundauflage kann es sich um eine primäre Wundauflage handeln, also um eine Wundauflage, die direkt auf der zu behandelnden Wunde aufgebracht wird, oder um eine sekundäre Wundauflage, die über einer getrennt davon applizierten Wundauflage auf die Wunde gebracht wird. Eine geeignete Wundauflage kann eine einzelne Wundkontaktschicht umfassen oder aus mehreren Schichten zusammengesetzt sein. Diese Schichten können unterschiedliche Funktionen aufweisen.

In einer bevorzugten Ausführungsform enthält eine erfindungsgemäße Wundauflage eine Wundkontaktschicht. In einer weiteren bevorzugten Ausführungsform besteht eine erfindungsgemäße Wundauflage aus einer Wundkontaktschicht.

Als Wundkontaktschicht kann gemäß der vorliegenden Erfindung ein Material Verwendung finden, das keinen negativen Einfluss auf die Wundheilung ausübt. Hierbei steht eine Wundkontaktschicht bei Verwendung der erfindungsgemäßen Wundauflage in direktem Kontakt mit der Wunde. Diese Wundkontaktschicht kann einzig und allein dazu dienen, dass eine absorbierende Schicht von der zu behandelnden Wunde beabstandet wird, als auch dass diese Wundkontaktschicht weitere Funktionen in Bezug auf die Wundauflage als auch auf die zu behandelnde Wunde ausübt. Insbesondere kann eine erfindungsgemäße Wundauflage eine Wundkontaktschicht mit einer ersten Seite und einer zweiten Seite umfassen, wobei die Wundkontaktschicht eine Hydrogelmatrix, einen Polymerfilm, eine Hydrokolloidmatrix, ein Polymernetz, ein Nonwoven, ein Gewebe, ein Gewirk, ein Gestrick oder ein Adhäsiv umfasst.

Gemäß einer Weiterbildung der erfindungsgemäßen Wundauflage kann vorgesehen sein, dass die Wundkontaktschicht eine Vielzahl an Kanälen, Öffnungen oder Löcher zum Durchtritt von Flüssigkeiten umfasst. Insbesondere ist dabei vorgesehen, das die Wundkontaktschicht Kanäle aufweist, die einen Durchtritt von Wundsekret von der ersten Seite zur zweiten Seite bilden. In dieser Ausführungsform ist vorgesehen, dass die erste Seite der Wundkontaktschicht einen direkten Kontakt zu einer zu behandelnden Wunde und die zweite Seite der Wundkontaktschicht einen direkten Kontakt mit der ersten Seite der absorbierenden Schicht aufweist.

Gemäß einer Weiterbildung der Erfindung kann auch vorgesehen sein, dass die Wundkontaktschicht Kanäle, Öffnungen oder Löcher aufweist, die einen Durchmesser von 0,5 bis 5 mm aufweisen. Insbesondere weist die Wundkontaktschicht Kanäle, Öffnungen oder Löcher auf, die einen Durchmesser von 1 bis 3 mm aufweisen. Ganz besonders bevorzugt weist die Wundkontaktschicht auf der im anwendungsgerechten Zustand der Wundauflage der Wunde zugewandten, ersten Seite Öffnungen auf, die einen Durchmesser von 1 bis 3 mm aufweisen, wobei die zweite Seite der Wundkontaktschicht in direktem Kontakt mit einer absorbierenden Schicht steht.

Weiterhin bevorzugt kann die Wundkontaktschicht eine Vielzahl an Kanälen, Öffnungen oder Löcher zum Durchtritt von Flüssigkeiten aufweisen, wobei die Kanäle, Öffnungen oder Löcher an der ersten Seite der Wundkontaktschicht eine Fläche von höchstens 95 % der Fläche der ersten Seite der Wundkontaktschicht einnehmen. Hierbei ist weiterhin bevorzugt vorgesehen, dass die Kanäle, Öffnungen oder Löcher eine Fläche von höchstens 70 %, insbesondere höchstens 50 %, insbesondere höchstens 40 % und ganz besonders bevorzugt von höchstens 30 % der Fläche der ersten Seite der Wundkontaktschicht einnehmen. Ganz besonders bevorzugt weist die Wundkontaktschicht Kanäle, Öffnungen oder Löcher auf, die an der ersten Seite der Schicht eine Fläche von mindestens 5 % und höchstens 30 % der Fläche der ersten Seite der Wundkontaktschicht einnehmen. Ganz besonders weist die Wundauflage eine Wundkontaktschicht auf, die 2 bis 8 Löcher pro cm² aufweist. Hierbei kann vorgesehen sein, dass die Wundkontaktschicht ein gelochter Polymerfilm oder ein Polymernetz, insbesondere ein Polyurethanfilm oder ein Polymernetz ist.

Gemäß einer weiteren Ausführungsform der vorliegenden Erfindung kann weiterhin vorgesehen sein, dass eine erfindungsgemäße Wundauflage als Wundkontaktschicht eine Hydrokolloidmatrix umfasst. Diese Hydrokolloidmatrix kann aus einer klebenden Polymermatrix bestehen, in die Hydrokolloidpartikel dispergiert sind. Gemäß der vorliegenden Erfindung soll unter einem Hydrokolloid ein Material verstanden sein, das ein hydrophiles synthetisches oder natürliches Polymermaterial ist, das löslich oder absorbierend und/ oder quellend in Wasser ist. Vorzugsweise umfasst eine Wundkontaktschicht ein Hydrokolloid aus einem synthetischen oder natürlichen Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate wie Celluloseether oder Celluloseester, vernetzte oder nicht vernetzte Carboxyalkylcellulose oder Hydroxyalkylcellulose, Polyvinylalkohol, Polyvinylpyrrolidon, Agar, Guargum, Gelatine oder Mischungen hiervon.

Das Hydrokolloid kann sowohl in Form von Fasern als auch in Form von Partikeln und/ oder Fasern innerhalb einer Matrix vorliegen. Insbesondere kann das Hydrokolloid in Form von Partikeln in einer klebenden Polymermatrix vorliegen. Die klebende Polymermatrix umfasst dabei mindestens ein Co-Blockpolymer ausgewählt aus der Gruppe der AB-Diblock-Copolymere und/ oder ABA-Triblock-Copolymere, das aus den Monomeren Styrol, Butadien und Isopren aufgebaut ist. Der Anteil an Hydrokolloidpartikel in der Wundkontaktschicht kann vorzugsweise 10 bis 70 Gew.-% bezogen auf das Gesamtgewicht der Wundkontaktschicht aufweisen. Eine solche Zusammensetzung ist zum Beispiel mit der EP 1 007 597 B1 bekannt.

Gemäß einer weiteren Ausführungsform der vorliegenden Erfindung kann weiterhin vorgesehen sein, dass eine erfindungsgemäße Wundauflage als Wundkontaktschicht eine wasserhaltige Hydrogelmatrix umfasst.

Geeignete Hydrogelmatrices können gelbildende Polymere umfassen wie beispielsweise Methylcellulose, Ethylcellulose, Hydroxyethylcellulose, Hydroxypropylmethylcellulose, Carboxymethylcellulose und ihr Natriumsalz, Hydroxypropylcellulose, Polyacrylate, Polymere von Vinylalkoholen, Vinylestern, Vinylethern und von Carboxyvinylmonomeren, Polyvinylpyrrolidon, Polyacrylamide, Polyethylenglykole, Polypropylenglykole, Copolymere aus Polyethylenglykol und Polypropylenglykol, Polystyrene Polymere der Acrylamidomethylpropansulfonsäure und ihrer Salze, Alginate, Hyaluronsäure, sowie deren Mischungen umfassen. Geeignete Hydrogele sind beispielsweise in den veröffentlichten Patentanmeldungen WO00/45864 und DE102005035879 beschrieben.

In einer bevorzugten Ausführungsform enthält eine erfindungsgemäße Wundauflage eine absorbierende Schicht. In einer weiteren bevorzugten Ausführungsform besteht eine erfindungsgemäße Wundauflage aus einer absorbierenden Schicht.

Unter einer absorbierenden Schicht wird eine Schicht verstanden, die in der Lage ist, Flüssigkeit, bevorzugt Wundexsudat, aufzunehmen und zu speichern. Bevorzugt verwendet werden Lagen, die ein Flüssigkeitsaufnahmevermögen von mindestens 1g/g, stärker bevorzugt von mindestens 2g/g, stärker bevorzugt von mindestens 5g/g, besonders bevorzugt von mindestens 10 g/g, gemessen nach DIN-EN 13726-1 (2002).

Eine absorbierende Schicht kann einen Schaumstoff, ein Gittertüll, ein Gewebe, ein Gewirk, ein Gestrick, einen Vliesstoff oder ein Fasermaterial enthalten.

Als Schaumstoffe werden üblicherweise Werkstoffe mit über die gesamte Masse verteilten Zellen (offen, geschlossen oder beides) verstanden. Solche Werkstoffe weisen üblicherweise somit eine Rohdichte (gemäß DIN EN ISO 845) auf, die niedriger ist als die Dichte der Gerüstsubstanz. So kann die Dichte eines Schaumstoffes, bevorzugt eines Polyurethanschaumstoffes, beispielsweise zwischen 10 und 110 kg/m³, bevorzugt 15 bis 100 kg/m³, mehr bevorzugt 20 bis 95 kg/m³ aufweisen.

In einer weiteren Ausführung der Erfindung ist es weiterhin bevorzugt, Silikonschaumstoffe, z.B. mit einer Dichte von 50 bis 300 kg/m³ einzusetzen.

Bevorzugt wird ein offenzelliger Schaumstoff als Wundauflage (a) verwendet. Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Schaumstoffs wird üblicherweise nach ASTM D 2856-87, Verfahren B) bestimmt.

Ein geeigneter Schaumstoff kann einen Gradienten der Porengröße über die Dicke des Schaumstoffs aufweisen. Die Porengröße beträgt bevorzugt 1 µm bis 1000 µm, mehr bevorzugt 50 µm bis 800 µm. Die Bestimmung erfolgte mikroskopisch, wobei ein Probenquerschnitt mikroskopiert wurde; die angegebene Porengröße entspricht dem Mittelwert von 5 zufällig ausgewählten und ausgemessenen Poren an der Querschnittsoberfläche pro Probe.

Gittertüll besteht vorzugsweise aus einem hydrophoben Material, beispielsweise Polyester.

Gewöhnlicherweise werden unter Gewebe Webereierzeugnisse verstanden. Hierzu zählen beispielsweise Tuch, Samt und sonstige textile Flächengebilde aus speziellen Fadenanordnungen, welche im Wesentlichen senkrecht zueinander sind. Die Fäden in Längsrichtung werden Kettfäden genannt und die Fäden in Querrichtung heißen Schussfäden. Um eine ausreichende Festigkeit des Gewebes zu erreichen, müssen die Kett- und Schussfäden dicht miteinander verwoben werden und zeigen deshalb ein geschlossenes Erscheinungsbild.

Gewirke sind auch unter dem Begriff Gewirk oder Wirkwaren bekannt. Gewirke gehören zu den Maschenwaren und sind aus Fadensystemen durch Maschenbildung meist maschinell hergestellte Stoffe.

Unter einem Vliesstoff soll ein Flächen- oder Raumgebilde aus gerichtet angeordneten oder wahllos zueinander befindlichen Fasern verstanden werden, welche mechanisch und/oder thermisch und/oder chemisch verfestigt wurden. Die Vliesstoffe (im Englischem auch "nonwoven") sind wesentlich verschieden von Geweben, Gestricken und Gewirken.

Die Vliesstoffe der vorliegenden Erfindung können Fasern natürlichen oder synthetischen Ursprungs oder Gemische davon enthalten. Zu den Fasern natürlichen Ursprungs zählen z.B. Seide, Cellulose, Baumwolle und Wolle. Die Fasern synthetischen Ursprungs umfassen die synthetischen Polymere (Kunstfasern) wie Viskose, Polyacrylate, Polyamide, Polyimide, Polyamidimide, Polyurethane, Polyester (insbesondere Polyethylenterephthalate und Polybutylenterephthalate), Polyetherester, Polyether, Polyacrylnitrile, Polyalkene (insbesondere Polyethylene und Polypropylene) Polyurethane und Polytetrafluorethylene.

Das Fasermaterial ist bevorzugt ein hydrophiles Fasermaterial. Hierbei können Fasern aus Cellulose, bevorzugt wasserunlösliche Fasern aus Cellulose, insbesondere weitgehend delignifizierte technische Zellstofffasern, insbesondere Holzstofffasern verwendet werden. Insbesondere können Fasern mit einer Faserlänge von < 5 mm Verwendung finden. Das Fasermaterial kann auch hydrophiles Fasermaterial aus regenerierter Cellulose, Carboxymethylcellulose, Carboxyethylcellulose, Hydroxymethylcellulose oder Hydroxyethylcellulose enthalten. Es kann auch vorgesehen sein eine Fasermischung aus Cellulose-, regenerierter Cellulose-, Carboxymethylcellulose-, Carboxyethylcellulose-, Hydroxymethylcellulose- oder Hydroxyethylcellulose-Fasern und Fasern aus Polyethylen, Polypropylen oder Polyester zu verwenden.

Die absorbierende Schicht kann zusätzlich ein Partikelgemisch aus Polyacrylatpartikeln enthalten, wobei die Polyacrylatpartikel ein vernetztes und/oder quervernetztes Polyacrylat umfassen.

Es kann jedoch auch vorgesehen sein, dass zwischen der absorbierenden Schicht und der Wundkontaktschicht eine Übergangsschicht angeordnet ist. Eine derartige Übergangsschicht kann dadurch entstehen, dass Teile der Wundkontaktschicht mit Teilen der absorbierenden Schicht vermischt werden. So entsteht eine besonders stabile Verbindung zwischen Wundkontaktschicht und absorbierender Schicht. Eine Übergangsschicht kann auch in einer Lage einer weiteren flüssigkeitsdurchlässigen Materialschicht sein, die sowohl mit der Wundkontaktschicht als auch mit der absorbierenden Schicht verbunden ist. In diesem Fall sorgt die Übergangsschicht für einen schnellen und gleichmäßigen Transport anfallenden Wundexsudats von der Wundoberfläche zur absorbierenden Schicht.

Gemäß einem weiteren weiterführenden Gedanken der vorliegenden Erfindung, ist auch eine Wundauflage Gegenstand der vorliegenden Erfindung, die zwischen der Hydrogelmatrix und dem hydrophilen Polymerschaum eine Barriereschicht aufweist. Eine solche Barriereschicht kann beispielsweise einen Polymerfilm umfassen, der mit Öffnungen versehen ist.

Weiterhin kann eine erfindungsgemäße Wundauflage eine Trägerschicht umfassen. Diese Trägerschicht kann aus verschiedenen Materialien bestehen. Üblicherweise werden in Wundauflagen textile Trägermaterialien, Nonwoven, Polymerfilme oder Polymerschäume eingesetzt. Diese Trägerschicht kann direkten Kontakt oder indirekten Kontakt zu der zweiten Seite der absorbierenden Schicht oder dem hydrophilen Polymerschaum aufweisen. Bei einem direkten Kontakt wird die Trägerschicht direkt auf die absorbierende Lage oder den Polyurethanschaum auflaminiert, wogegen bei einem indirekten Kontakt die Trägerschicht mittels eines Adhäsivs auf die absorbierende Schicht oder dem Polyurethanschaum aufgebracht ist. Dabei kann das Adhäsiv vollflächig oder lediglich in Teilbereichen zwischen der Trägerschicht und der absorbierenden Schicht aufgebracht sein.

Als Trägerschicht einer erfindungsgemäßen Wundauflage können insbesondere Polymerfilme oder Polymerschäume eingesetzt werden. Ganz besonders bevorzugt sind Polymerfilme oder Polymerschäume, die wasserundurchlässig sind und die eine hohe Wasserdampfdurchlässigkeit aufweisen. Hierzu sind besonders Filme oder Schäume geeignet, die aus Polyurethan, Polyetherurethan, Polyesterurethan, Polyether-Polyamid-Copolymeren, Polyacrylat oder Polymethacrylat gefertigt werden. Insbesondere ist als Trägerschicht ein wasserundurchlässigen und wasserdampfdurchlässigen Polyurethanfilm oder einen wasserundurchlässigen und wasserdampfdurchlässigen Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen. Die Wasserdampfdurchlässigkeit des Polymerfilms der Wundauflage weist vorzugsweise mindestens 750 g/ m²/ 24 Std., insbesondere mindestens 1000 g/ m2/ 24 Std. und ganz besonders bevorzugt mindestens 2000 g/ m²/ 24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Darüber hinaus ist sichergestellt, dass keine Flüssigkeit zwischen der Folie und der die zu behandelnden Fläche umgebenden Haut austreten kann. Als besonders bevorzugt sind solche Klebstoffe zu betrachten, die in einem dünnen Auftrag von 20 bis 35 g/ m² zusammen mit dem Film eine Wasserdampfdurchlässigkeit von mindestens 800 g/ m²/ 24 Std. und vorzugsweise von mindestens 1000 g/ m²/ 24 Std. (gemessen nach DIN EN 13726) aufweisen.

Unter einer Brönsted-Säure wird eine Substanz verstanden, die in Reaktion mit geeigneten Lösemitteln Wasserstoffkationen (Protonen) abgeben kann. Dabei entstehen als Reaktionsprodukte ein protoniertes Lösemittelteilchen und ein sogenannter Säurerest, der zur Brönstedsäure die korrespondierende bzw. konjugierte Base darstellt. Stoffgemische, die sowohl Brönstedsäure als auch korrespondierende Base enthalten, werden als Puffersubstanzen bezeichnet. Diese Gemische zeichnen sich dadurch aus, dass sie in wässriger Lösung den pH-Wert der Lösung stabilisieren, selbst wenn zu der wässrigen Lösung Mengen einer Säure oder einer Base zugegeben werden. Eine zur wässrigen Lösung zugegebene Säure reagiert mit der in der Puffersubstanz vorhandenen Base unter Ausbildung eines neuen Teilchens der zur Base korrespondierenden Brönstedsäure. Es entstehen keine weiteren Hydroxonium- oder Hydroxidionen, so dass der pH-Wert beinahe unverändert bleibt. Diese pH-Wert-Stabilisierung funktioniert, so lange die Menge an enthaltenen Basen- oder Säureteilchen nicht übersteigt. Das quantitative Ausmaß dieser Stabilisierung wird als Pufferkapazität bezeichnet.

Erfindungsgemäße Wundauflagen können sowohl eine Brönstedsäure alleine als auch eine Mischung aus Brönstedsäure und ihrer korrespondierenden Base enthalten. Wenn eine Brönstedsäure gemeinsam mit ihrer korrespondierenden Base eingesetzt wird, besteht das molare Verhältnis aus Brönstedsäure und ihrer korrespondierenden Base 100:1 bis 1:100, bevorzugt 10:1 bis 1:10, besonders bevorzugt 1:1. Eine äquimolare Mischung aus Brönstedsäure und ihrer korrespondierenden Base besitzt die größte pH-Wert-Stabilität gegenüber Säure- oder Basenzugabe.

In einer bevorzugten Ausführungsform enthält eine erfindungsgemäße Wundauflage eine Brönstedsäure und ein pH-sensitives Polymer, worin die Brönstedsäure in eine Matrix aus pH-sensitivem Polymer eingebettet ist oder von pH-sensitivem Polymer umgeben ist, und zusätzlich eine Substanz, die eine zu der Brönstedsäure korrespondierende Brönstedbase darstellt und die gemeinsam mit der Brönstedsäure in eine Matrix aus pH-sensitivem Polymer eingebettet ist oder von pH-sensitivem Polymer umhüllt ist.

Erfindungsgemäß werden Brönstedsäuren eingesetzt, die aus der Gruppe der Stoffe Essigsäure, Zitronensäure, Milchsäure, Glycerinsäure, Gluconsäure, Benzoesäure, Aconitsäure, Glutarsäure, Weinsäure, Phosphorsäure, Äpfelsäure, Bernsteinsäure, Glutaminsäure ausgewählt sind. Diese Brönstedsäuren weisen einen vorteilhaften pKₛ-Wert zwischen 2 und 5 auf, sind physiologisch unbedenklich und können in der Wundheilung eingesetzt werden.

Besonders bevorzugt werden Zitronensäure, Benzoesäure und/oder Milchsäure als Brönstedsäuren verwendet, gegebenenfalls gemeinsam mit ihren korrespondierenden Basen. Diese Brönstedsäuren weisen eine besonders gute Gewebeverträglichkeit auf.

Erfindungsgemäße Wundauflagen enthalten eine oder mehrere Brönstedsäuren in einer oder mehrerer voneinander verschiedenen Schichten oder Lagen. Dabei enthält jede eine Brönstedsäure enthaltende Lage pro Gramm 0,01-100mmol Brönstedsäure, bevorzugt 0,1-10mmol Brönstedsäure, besonders bevorzugt 0,2-5mmol Brönstedsäure. Dabei kann eine oder mehrere Brönstedsäuren, gegebenenfalls gemeinsam mit ihrer korrespondierenden Base, in einer Wundkontaktschicht, einer absorbierenden Schicht, einer Übergangsschicht, einer Transportschicht, einer Stützschicht oder einer Rückschicht enthalten sein. Es hat sich gezeigt, dass mit einem derartigen Gehalt an Brönstedsäure eine besonders vorteilhafte Beeinflussung des pH-Werts des anfallenden Wundexsudats erzielt werden kann.

Im Rahmen der vorliegenden Erfindung wird unter einem pH-sensitiven Polymer eine Substanz oder ein Substanzgemisch verstanden, welches aus einer Vielzahl sich wiederholender Monomereinheiten aufgebaut ist und das in einer wässrigen Lösung bei einer bestimmten Temperatur und einem ersten pH-Wert eine erste Löslichkeit bzw. Lösungsgeschwindigkeit aufweist und bei derselben Temperatur und einem zweiten pH-Wert, der vom ersten pH-Wert verschieden ist, eine zweite Löslichkeit bzw. Lösungsgeschwindigkeit, die von der ersten Löslichkeit bzw. Lösungsgeschwindigkeit verschieden ist. Daraus ergeben sich unterschiedliche Löslichkeiten bzw. Lösungsgeschwindigkeiten des Polymers bei verschiedenen pH-Werten. Geeignet sind Polymere, die bei einem pH-Wert von weniger al 6,0 in wässriger Lösung nicht löslich und bei einem pH-Wert von mehr al 7,0 in einer Menge von wenigstens 1% (w/w) löslich sind. Bevorzugt geeignet sind Polymere, die bei einem pH-Wert von weniger als 6,0 in wässriger Lösung bei 20°C eine Lösungsgeschwindigkeit von weniger als 10mg/min/g aufweisen, bevorzugt weniger als 1mg/min/g und bei einem pH-Wert von mindestens 8,0 in wässriger Lösung bei 20°C eine Lösungsgeschwindigkeit von mehr als 50mg/min/g, bevorzugt mehr als 100mg/min/g aufweisen. Geeignete Polymere sind Celluloseacetatphthalat, Celluloseacetattrimellitat, Hydroxypropylmethylcelluloseacetatsuccinat, Carboxymethylethylcellulose, oxidierte regenerierte Cellulose, Polyacrylate, deren Copoylmere und Mischungen. Erfindungsgemäße pH-sensitive Polymere sind Copolymerisate der Methacrylsäure mit Methacrylsäuremethylester, also ein Methacrylsäure-comethylmethacrylat, bevorzugt im Verhältnis 1:1 bis 1:2. Derartige Polymere sind beispielsweise unter dem Namen Eudragit^{®} (Evonik, Darmstadt, Deutschland) erhältlich. Bevorzugte Polymerzubereitungen sind unter den Namen Eudragit^{®} S 100, Eudragit^{®} S 12,5, Eudragit FS 30 D, Eudragit^{®} L 100, Eudragit^{®} L 12,5 erhältlich. Auch Mischungen dieser Polymerzubereitungen können eingesetzt werden. Diese Polymere weisen besonders günstige pH-abhängige Lösungseigenschaften auf.

Bevorzugt betrifft die vorliegende Erfindung Wundauflagen, die Partikel enthält, welche eine Brönstedsäure und ein pH-sensitives Polymer umfassen. Partikel können in verschiedenen Lagen einer Wundauflage untergebracht werden, ohne dass die physikalischen Eigenschaften der jeweiligen Lage beeinträchtigt werden, wie beispielsweise Absorptionsvermögen, Elastizitätsmodul oder Quellvermögen.

Im Rahmen der vorliegenden Anmeldung werden unter dem Begriff Mikropartikel alle Partikel verstanden, ungeachtet ihrer Gestalt, deren Äquivalentdurchmesser im Größenordnungsbereich von 50nm bis 1000 µm liegt. Dabei bezieht sich der Unterbegriff Mikrosphärule auf sphärisch geformte Mikropartikel, während der Ausdruck Mikrokapsel solche Partikel beschreibt, bei denen eine äußere Polymerschicht einen flüssigen, gasförmigen oder festen Kern umhüllt. Partikel, in denen der Wirkstoff gleichmäßig, mehr oder weniger fein im Polymer verteilt vorliegt, bezeichnet man auch als Mikromatrices. Bevorzugt handelt es sich bei den verwendeten Partikeln um Mikropartikel, Nanopartikel oder Liposomen.

Mikropartikel sind Partikel mit einem Partikeldurchmesser zwischen 1µm und 1000µm. Man unterscheidet Mikrokapseln und Mikrosphärulen.

Mikrokapseln enthalten einen festen oder flüssigen Kern, der von einem Polymer als Wandmaterial umgeben ist. Gemäß der vorliegenden Erfindung hergestellte Wundversorgungsprodukte können Mikrokapseln enthalten, bei denen Brönstedsäuren und gegebenenfalls ihre korrespondierenden Basen in fester und/oder flüssiger und/oder gelöster Form im Kern enthalten sind und von einem Polymer als Wandmaterial umgeben sind.

In einer bevorzugten Ausführungsform enthält eine erfindungsgemäße Wundauflage Partikel, insbesondere Mikropartikel, die einen Kern aufweisen, der eine Brönstedsäure und gegebenenfalls ihre korrespondierende Base enthält und der von einem pH-sensitiven Polymer umhüllt ist.

Mikrosphärulen sind Partikel, bei denen eine Puffersubstanz in einer Polymermatrix ohne Ausbildung einer gesonderten Kapselwand eingebettet ist. In einer bevorzugten Ausführungsform enthält eine erfindungsgemäße Wundauflage Partikel, die eine aus einem pH-sensitiven Polymer gebildete Matrix umfassen, in die eine Brönstedsäure und gegebenenfalls ihre korrespondierende Base eingebettet ist.

Nanopartikel sind kolloidale Feststoffsysteme mit einem Durchmesser von 50-1000nm. Es wird zwischen Nanokapseln und Nanosphärulen unterschieden.

Nanokapseln sind verfestigte mizellare Systeme, verfestigte Mikroemulsionen oder umhüllte kolloidale Feststoffsysteme.

Nanosphärulen werden auch als Nanopellets bezeichnet und sind kolloidale Teilchen, bei denen ein Stoff in eine Polymermatrix eingebettet ist.

Liposomen sind kolloidale kugelförmige Lipidvesikeln mit wässrigem Kern und einem Durchmesser von 20nm bis 3 µm.

Bevorzugt werden Partikel mit einem Äquivalentdurchmesser von 5µm-300µm verwendet. Derartige Mikropartikel sind in der Lage, auch in feine Öffnungen des Materials der Lage, in der die Partikel eingebracht werden, einzudringen und so gleichmäßig in der jeweiligen Lage verteilt vorzuliegen. Aufgrund des geringen Äquivalenzdurchmessers wird eine hohe Oberfläche der Gesamtheit der Brönstedsäure-Polymerpartikel erreicht, so dass anfallendes Wundexsudat unmittelbar in innigen Kontakt mit Brönstedsäure-haltigen Partikeln kommt, so dass bei Bedarf sofort eine ausreichende Menge an Brönstedsäure freigesetzt werden kann.

Der Äquivalentdurchmesser kann mittels Laserbeugung bestimmt werden, beispielsweise mit einem Mastersizer 2000 der Firma Malvern. Die Auswertung erfolgt bevorzugt nach der Fraunhofer-Methode.

Geeignete Partikel können in verschiedenen Verfahren hergestellt werden. Grundsätzlich sind dem Fachmann zahlreiche Verfahren zur Herstellung von Partikeln bekannt, in denen eine Substanz mit einem geeigneten polymeren Material kombiniert wird. So wird beispielsweise auf das Lehrbuch der Pharmazeutischen Technologie der Autoren Bauer, Frömming und Führer hingewiesen, welches 1999 in der sechsten Auflage bei der Wissenschaftlichen Verlagsgesellschaft Stuttgart erschienen ist.

Einige geeignete Herstellungsverfahren werden im Folgenden dargestellt.

Bei den Lösemittelextraktions/-evaporationsverfahren werden Emulsionströpfchen einer organischen Polymerlösung, in die eine Brönstedsäure und gegebenenfalls ihre korrespondierende Base auf verschiedene Weise eingearbeitet sein können, in eine Flüssigkeit eingebracht, in der das organische Lösemittel der dispersen Phase löslich ist und dadurch aus den Tröpfchen extrahiert wird. Die Brönstedsäure und gegebenenfalls ihre korrespondierende Base kann entweder gemeinsam mit dem Polymer in der dispersen Phase gelöst oder in fein pulverisierter Form in dieser dispergiert werden. Ebenso können wässrige Lösungen einer Brönstedsäure und gegebenenfalls ihrer korrespondierenden Base in der dispersen Phase emulgiert werden, wodurch sich mit der kontinuierlichen, äußeren Phase eine Doppelemulsion bildet. Die Tröpfchenbildung erfolgt mit Hilfe von Rührwerkzeugen oder statischen Mischern. Dieser Prozessschritt bestimmt maßgeblich die mittlere Partikelgröße und die Partikelgrößenverteilung des jeweiligen Endproduktes. Im einfachsten Fall wird die äußere Phase in einem Mischgefäß vorgelegt und bei ausreichender Rührgeschwindigkeit die säurehaltige Polymerlösung zugegeben. Auch die umgekehrte Vorgehensweise, also die Zugabe der äußeren Phase in die gerührte Polymerlösung ist möglich, wobei in diesem Fall die Tröpfchenbildung unter Phaseninversion erfolgt. Durch Entzug des Polymer-Lösemittels werden die gebildeten Tröpfchen ausgehärtet und in Mikrosphärulen überführt. Bei ausreichender Löslichkeit des verwendeten Lösemittels in der äußeren Phase oder bei deren ausreichend hohem Überschuss erfolgt der Lösemittelentzug aus den Emulsionströpfchen allein durch einen Extraktionsprozess. Wenn das Verteilungsgleichgewicht jedoch nicht genügend weit auf der Seite des Extraktionsmittels liegt, muss der Vorgang durch kontinuierlichen Entzug des Polymerlösemittels beispielsweise durch Evaporation oder Dialyse unterstützt werden. Im Hinblick auf eine hohe Einschlusseffizienz werden vorzugsweise solche Extraktionsflüssigkeiten verwendet, in denen die Brönstedsäure nicht oder nur schwerlöslich ist. Die Geschwindigkeit des Lösungsmittelentzugs ist ein wesentlicher Einflussfaktor, der genutzt werden kann, um die innere Struktur und mit dieser das Freisetzungsverhalten der Mikropartikel gezielt zu steuern. Nach dem Extraktions-/Evaporationsprozess werden die entstandenen Mikropartikel abfiltriert oder abzentrifugiert, gewaschen und unter materialschonenden Bedingungen getrocknet.

Beim Phasenseparationsverfahren wird die Brönstedsäure und gegebenenfalls ihre korrespondierende Base in wässriger Lösung oder als mikronisierte Festsubstanz in einer organischen Polymerlösung, beispielsweise Dichlormethan, Ethylacetat, Acetonitril emulgiert bzw. suspendiert. Durch Zugabe eines Phasentrennmittels, beispielsweise Silikonöl, kommt es zur Koazervation des Polymers. Durch Adsorption des Koazervates an die Oberfläche der wässrigen Tröpfchen bzw. der festen Säurepartikel, werden diese von einer zunächst noch sehr weichen Polymerhülle überzogen. Durch Auswaschen des Phasentrennmittels durch ein Nichtlösemittel für das Polymer, beispielsweise Hexan oder Heptan, wird die Hülle gehärtet, wonach die entstandenen Mikrokapseln abgetrennt werden können.

Beim Sprühtrocknungsverfahren wird eine gemeinsame Lösung von Brönstedsäure, gegebenenfalls ihrer korrespondierenden Base und Polymer in einem organischen Lösemittel oder eine Suspension der Brönstedsäure und gegebenenfalls ihrer korrespondierenden Base in der organischen Polymerlösung in einen heißen Luftstrom hinein zerstäubt. Das trockene Produkt wird durch einen Zyklon aus dem Luftstrom abgetrennt. Mit diesem Verfahren werden Partikel erhalten, bei denen eine Brönstedsäure und gegebenenfalls ihre korrespondierende Base in einer Matrix aus pH-sensitivem Polymer eingebettet sind. Derartige Partikel können besonders kostengünstig hergestellt werden.

In einem weiteren geeigneten Herstellungsverfahren wird eine Brönstedsäure und gegebenenfalls ihre korrespondierende Base in Pulverform in einen Dragierkessel gegeben und mit einer geeigneten Polymerlösung oder Polymerdispersion besprüht. Es entstehen Mikrokapseln.

In einem weiteren geeigneten Herstellungsverfahren wird eine Brönstedsäure und gegebenenfalls ihre korrespondierende Base in Pulverform in der Wirbelschicht mit einer geeigneten Polymerlösung oder Polymerdispersion besprüht. Ein besonders geeignetes Verfahren ist das Wurster-Verfahren.

In einem weiteren geeigneten Verfahren wird eine Puffersubstanz zu einer Lösung eines geeigneten Monomers gegeben und das Monomer zur Polymerisation veranlasst. Dabei entstehen Partikel, in denen die Puffersubstanz in eine polymere Matrix eingebettet sind.

Im Rahmen der vorliegenden Erfindung können eingesetzte Partikel ein Massenverhältnis von Brönstedsäure zu Polymer zwischen 100:1 und 1:100 aufweisen, bevorzugt zwischen 10:1 und 1:10, besonders bevorzugt zwischen 10:1 und 5:1. Aus derartigen Partikeln wird die enthaltene Brönstedsäure bei Kontakt mit Wundexsudat mit erhöhtem pH-Wert mit einer besonders vorteilhaften Geschwindigkeit freigesetzt.

Die vorliegende Erfindung offenbart auch Partikeln, die eine Brönstedsäure, gegebenenfalls ihre korrespondierende Base und ein pH-sensitives Polymer umfassen, zur Verwendung in der Behandlung von Wunden, bevorzugt infizierter Wunden und/oder chronischer Wunden.

Die Behandlung der Wunden, bevorzugt der sekundär heilenden Wunden, kann eine phasengerechte Wundbehandlung sein. Unter einer phasengerechten Wundtherapie wird im Rahmen dieser Anmeldung verstanden, dass die Wundtherapie auf die spezifischen Bedürfnisse der Wunde in den einzelnen Phasen eingeht.

So kann die phasengerechte Behandlung gezielt in einer oder mehreren Phasen der Wundheilung erfolgen (Im Gegensatz dazu erfolgt bei herkömmlichen Wundbehandlungen ein und dieselbe Behandlung über alle Phasen). So werden die Partikel bevorzugt in der Reinigungsphase und/oder in der Granulationsphase verwendet, da in diesen Phasen die häufigsten Störungen des pH-Milieus auftreten.

Eine erfindungsgemäße Wundauflage kann eine Brönstedsäure und gegebenenfalls ihre korrespondierende Base in einer Wundkontaktschicht enthalten. Die Wundkontaktschicht kann ein flüssigkeitsdurchlässiges Material umfassen. Geeignete Materialien sind textile Materialien wie Vliese, Gewebe, Gestricke oder Gewirke, oder nicht textile Materialien wie perforierte Folien, Kunststoffnetze oder Ähnliches. Dieses flüssigkeitsdurchlässige Material kann zunächst mit einer Lösung einer Brönstedsäure und gegebenenfalls ihrer korrespondierenden Base imprägniert und anschließend vollständig getrocknet werden, so dass die Brönstedsäure und gegebenenfalls ihre korrespondierende Base vollständig im entsprechenden Material enthalten ist. Anschließend kann das so erhaltene Material in eine Lösung oder Dispersion eines geeigneten pH-sensitiven Polymers getaucht werden, woraufhin das Löse- oder Dispersionsmittel verdampft wird. Ebenfalls möglich ist, das erhaltene Material mit einer Lösung oder Dispersion eines geeigneten pH-sensitiven Polymers zu besprühen und anschließend zu trocknen. In beiden Fällen wird eine gleichmäßige Beschichtung des mit einer Brönstedsäure und gegebenenfalls ihrer korrespondierenden Base imprägnierten Materials erzielt. Um eine höhere Schichtdicke zu erzielen, kann der geschilderte Beschichtungsvorgang mehrfach wiederholt werden. Bevorzugt werden Materialien, die ein bis zehnmal in der beschriebenen Weise mit einer Schicht aus pH-sensitivem Polymer beschichtet werden. Alternativ kann das Material mit einer wässrigen Suspension, die Mikropartikel aus Brönstedsäure, gegebenenfalls ihrer korrespondierenden Base und pH-sensitivem Polymer enthalten, imprägniert oder besprüht werden. Anschließend wird das Material wiederum vollständig getrocknet, so dass die Mikropartikel im Material verbleiben. Falls es sich bei der Wundkontaktschicht um eine halbfeste Zubereitung wie beispielsweise eine Salbe, Creme, Paste oder ein Gel, bevorzugt ein Hydrogel handelt, kann eine Dispersion aus Partikeln, die eine Brönstedsäure, gegebenenfalls ihre korrespondierende Base und ein pH-sensitives Polymer umfassen direkt in die vorgefertigte halbfeste Zubereitung eingearbeitet werden, beispielsweise durch Verrühren oder Verreiben. Eine Dispersion aus Partikeln, die eine Brönstedsäure, gegebenenfalls ihre korrespondierende Base und ein pH-sensitives Polymer umfassen, kann auch in einem der Bestandteile der halbfesten Zubereitung gelöst oder dispergiert werden und anschließend mit den übrigen notwendigen Bestandteilen zur fertigen halbfesten Zubereitung weiterverarbeitet werden.

Eine derartige Wundkontaktschicht besitzt den Vorteil, dass anfallendes Wundexsudat unmittelbar mit in der Wundkontaktschicht enthaltener Brönstedsäure in Berührung kommt, so dass ein ungünstig alkalischer pH-Wert des anfallenden Wundexsudats sofort korrigiert werden kann.

Eine derartige Wundkontaktschicht kann alleine als primäre Wundauflage verwendet werden, gemeinsam mit darauf aufgebrachten sekundären Wundauflagen oder als Schicht in einer mehrschichtigen Wundauflage enthalten sein.

Eine erfindungsgemäße Wundauflage kann eine absorbierende Schicht enthalten. Diese absorbierende Schicht kann zunächst mit einer Lösung einer Brönstedsäure und gegebenenfalls ihrer korrespondierenden Base imprägniert und anschließend vollständig getrocknet werden, so dass die Brönstedsäure und gegebenenfalls ihre korrespondierende Base vollständig im entsprechenden Material enthalten ist. Anschließend kann das so erhaltene Material in eine Lösung oder Dispersion eines geeigneten pH-sensitiven Polymers getaucht werden, woraufhin das Löse- oder Dispersionsmittel verdampft wird. Ebenfalls möglich ist, das erhaltene Material mit einer Lösung oder Dispersion eines geeigneten pH-sensitiven Polymers zu besprühen und anschließend zu trocknen. In beiden Fällen wird eine gleichmäßige Beschichtung des mit einer Brönstedsäure und gegebenenfalls ihrer korrespondierenden Base imprägnierten Materials erzielt. Um eine höhere Schichtdicke zu erzielen, kann der geschilderte Beschichtungsvorgang mehrfach wiederholt werden. Bevorzugt werden Materialien, die ein bis zehnmal in der beschriebenen Weise mit einer Schicht aus pH-sensitivem Polymer beschichtet werden.

Alternativ kann das Material mit einer wässrigen Suspension, die Mikropartikel aus Brönstedsäure, gegebenenfalls ihrer korrespondierenden Base und pH-sensitivem Polymer enthalten, imprägniert oder besprüht werden. Anschließend wird das Material wiederum vollständig getrocknet, so dass die Mikropartikel im Material verbleiben.

Eine derartige absorbierende Schicht kann große Mengen Brönstedsäure enthalten, so dass sie auch einen besonders hohen pH-Wert anfallenden Wundexsudats oder den pH-Wert großer Mengen ungünstig alkalischen Wundexsudats korrigieren kann.

Eine derartige absorbierende Schicht kann alleine als primäre Wundauflage verwendet werden, gemeinsam mit darauf aufgebrachten sekundären Wundauflagen oder als Schicht in einer mehrschichtigen Wundauflage enthalten sein.

### Beispiele:

### Beispiel 1: Umhüllte Wundkontaktschicht

Ein 10cm x 10cm messendes Polyamidgewirk mit einem Flächengewicht von 85g/m² wird mit 12,75mL einer Lösung aus 0.3mM Milchsäure imprägniert. Nach vollständiger Trocknung verbleibt die gesamte Milchsäure in dem Polyamidgewirk. Das so erhaltene Material wird mit einer handelsüblichen organischen Lösung Eudragit^{®} S 12,5 imprägniert, indem es für 10s in die Lösung getaucht wird. Das so imprägnierte Material wird getrocknet. Nach vollständiger Trocknung verbleibt eine komplett mit pH-sensitivem Polymer überzogene Wundkontaktschicht. Um eine ausreichende Schichtdicke zu erhalten, wird der Beschichtungsvorgang fünf Mal wiederholt.

### Beispiel 2: Umhüllte absorbierende Schicht

Ein Polyurethan-Schaum (Vivo MCF03, AMS) wurde pro Gramm Schaummaterial mit 15mL einer wässrigen Lösung enthaltend Benzoesäure und Natriumbenzoat, jeweils in einer Konzentration von 0,04mol/L versehen, um eine Stabilisierung des pH-Werts im pH-Bereich pH 4 bis pH 4,5 und eine Pufferkapazität von etwa 0,3 - 0,4 mmol NaOH zu erreichen. Nach der Imprägnierung wurde eine vollständige Trocknung des Polyurethan-Schaums vorgenommen. Das so erhaltene Produkt wurde mit einer handelsüblichen organischen Lösung des pH-sensitiven Polymers Eudragit S 12,5 (Polymethacrylsäure-comethylmethacrylat 1:2) imprägniert. Nach vollständiger Trocknung verbleibt ein mit Polyacrylat beschichteter Schaum. Um eine ausreichende Schichtdicke zu erhalten, wird der Beschichtungsvorgang noch einmal wiederholt.

### Beispiel 3: Herstellung von Mikropartikeln mittels Sprühtrocknung

Es wurden verschiedene Mikropartikel aus Milchsäure mit Polymergehalten von 10% bis 40% hergestellt. Dazu wurden jeweils 300ml einer Dispersion hergestellt, die anschließend mit Hilfe eines Büchi 190 Mini Spray Dryers sprühgetrocknet wurden. Die Zusammensetzungen der Dispersionen sind in folgender Tabelle aufgelistet:

| Nummer | % Polymer | Milchsäure | Eudragit FS 30 D (enthaltenes Polymer) | Wasser |
|---|---|---|---|---|
| MP01 | 10 | 54,0g | 20,0g (6,0g) | 226,0g |
| MP02 | 20 | 48,0g | 40,0g (12,0g) | 212,0g |
| MP03 | 30 | 42,0g | 60,0g (18,0g) | 198,0g |
| MP04 | 40 | 36,0g | 80,0g (24,0g) | 184,0g |

Die Sprühtrocknung erfolgte mit einem Büchi 190 Mini Spray Dryer (Büchi Laborato-riums-Technik, Eislingen Deutschland). Als Trocknungstemperatur wurde 95°C gewählt. Die Pumpleistung, mit der die Dispersion versprüht wurde, lag bei ca. 5 ml/min. Als Aspiratorleistung wurde eine Einstellung von 50% gewählt. Die Lufttemperatur betrug am Auslass 48°C.

Zur weiteren Verarbeitung wurde der erhaltene Rückstand entweder in Wasser suspendiert, so dass eine Suspension erhalten wurde, oder nach erneutem Verreiben in einer Reibeschale direkt in ein geeignetes Medium eingearbeitet.

### Beispiel 4: Mikropartikel in einer Wundkontaktschicht

Ein 10cm x 10cm messendes Polyamidgewirk mit einem Flächengewicht von 85g/m² wird mit 1,418g einer 30%igen Dispersion aus gemäß Beispiel 3 erhaltenen Milchsäure-Polymermikropartikeln mit einem Polymergehalt von 10% (MP01) imprägniert. Nach vollständiger Trocknung verbleiben die Milchsäure-Polymerpartikel in dem Polyamidgewirk.

### Beispiel 5: Mikropartikel in absorbierender Schicht

Ein Polyurethan-Schaum (Vivo MCF03, AMS) wurde pro Gramm Schaummaterial mit 7,5g einer 10%igen wässrigen Dispersion aus gemäß Beispiel 3 erhaltenen Milchsäure-Polymerpartikel mit einem Polymergehalt von 40% (MP04) imprägniert. Das so erhaltene Schaummaterial kann in feuchtem oder vollständig getrocknetem Zustand verwendet werden.

### Beispiel 6: Mikropartikel in Hydrogel

Eine Suspension wird hergestellt aus 30,0g Hydroxyethylcellulose, 225,2g gemäß Beispiel 3 hergestellter Milchsäure-Polymerpartikel mit einem Gehalt von 20% (MP02), und 200,0g Glycerin. In einem zweiten Schritt werden 7,0g Carbopol 980 NF (Polyacrylat; Fa. Lubrizol, Wickliffe, USA) mit 688,0g Ringer-Lösung versetzt und zwei Stunden bei Raumtemperatur gerührt. Unter Zugabe von ca. 75g Natronlauge der Konzentration 1 mol/L wird ein pH-Wert von 6,0 eingestellt und weitere zwei Stunden lang gerührt. In diese Lösung wird im Folgenden sehr langsam unter gleichmäßigem Rühren die im ersten Schritt hergestellte Suspension aus Hydroxyethylcellulose, Milchsäure-Polymerpartikeln und Glycerin gegeben. Nach der Zugabe wird weitere zwei Stunden lang gerührt. Eventuell eingearbeitete Luft kann anschließend durch Rühren im Vakuum entfernt werden. Es wird ein schwach saures Hydrogel erhalten, das pro Gramm 2,0mmol Milchsäure in mikroverkapselter Form enthält. Dieses Hydrogel kann direkt als amorphes Gel auf einer Wund appliziert werden oder als Wundkontaktschicht auf einer absorbierenden Schicht in einer mehrlagigen Wundauflage verwendet werden.

## Patentansprüche

1. Wundauflage enthaltend eine Brönstedsäure und ein pH-sensitives Polymer,
**dadurch gekennzeichnet, dass**
die Brönstedsäure in eine Matrix aus dem pH-sensitiven Polymer eingebettet ist oder von dem pH-sensitiven Polymer umhüllt ist,
wobei es sich bei der Brönstedsäure um eine Substanz ausgewählt aus der Gruppe Essigsäure, Zitronensäure, Milchsäure, Glycerinsäure, Gluconsäure, Benzoesäure, Aconitsäure, Glutarsäure, Weinsäure, Phosphorsäure, Äpfelsäure, Bernsteinsäure und Glutaminsäure handelt und
wobei es sich bei dem pH-sensitiven Polymer um ein Methacrylsäure-comethylmethacrylat-Polymer handelt.

2. Wundauflage gemäß Anspruch 1, **dadurch gekennzeichnet, dass** die Brönstedsäure in Partikeln enthalten ist, die die Brönstedsäure und das pH-sensitive Polymer umfassen.

3. Wundauflage gemäß Anspruch 2, **dadurch gekennzeichnet, dass** die Partikel einen Durchmesser von 50nm bis 1000µm, bevorzugt 5µm bis 300µm, aufweisen.

4. Wundauflage gemäß einem der Ansprüche 2 oder 3, **dadurch gekennzeichnet, dass** die Partikel einen Kern aufweisen, der die Brönstedsäure enthält und der von dem pH-sensitiven Polymer umhüllt ist.

5. Wundauflage gemäß einem der Ansprüche 2 oder 3, **dadurch gekennzeichnet, dass** die Partikel eine aus dem pH-sensitiven Polymer gebildete Matrix umfassen, in die die Brönstedsäure eingebettet ist.

6. Wundauflage nach einem der vorangehenden Ansprüche, **dadurch gekennzeichnet, dass** die Wundauflage zusätzlich eine Substanz enthält, die eine zur besagten Brönstedsäure korrespondierende Brönstedbase darstellt und die gemeinsam mit der Brönstedsäure in eine Matrix aus dem pH-sensitiven Polymer eingebettet ist oder von dem pH-sensitiven Polymer umhüllt ist.

7. Wundauflage gemäß einem der vorangehenden Ansprüche, **dadurch gekennzeichnet, dass** sie eine Wundkontaktschicht enthält oder aus einer Wundkontaktschicht besteht.

8. Wundauflage gemäß einem der vorangehenden Ansprüche, **dadurch gekennzeichnet, dass** sie eine absorbierende Schicht enthält oder aus einer absorbierenden Schicht besteht.

9. Wundauflage gemäß Anspruch 8, **dadurch gekennzeichnet, dass** die Brönstedsäure in der absorbierenden Schicht enthalten ist.

10. Wundauflage gemäß einem der Ansprüche 7 bis 9, **dadurch gekennzeichnet, dass** die Brönstedsäure in der Wundkontaktschicht enthalten ist.

11. Wundauflage gemäß einem der Ansprüche 7 bis 10, **dadurch gekennzeichnet, dass** die Brönstedsäure in einer Menge von 0,01-100mmol pro Gramm in einer jeweils die Brönstedsäure umfassenden Schicht enthalten ist.

## Claims

1. Wound dressing comprising a Bronsted acid and a pH-sensitive polymer, **characterized in that**
the Bronsted acid is embedded in a matrix composed of the pH-sensitive polymer or is encased in the pH-sensitive polymer,
wherein the Bronsted acid is a substance selected from the group comprising acetic acid, citric acid, lactic acid, glyceric acid, gluconic acid, benzoic acid, aconitic acid, glutaric acid, tartaric acid, phosphoric acid, malic acid, succinic acid and glutamic acid and
wherein the pH-sensitive polymer is a methacrylic acid-methyl methacrylate copolymer.

2. Wound dressing according to Claim 1, **characterized in that** the Bronsted acid is contained in particles that include the Bronsted acid and the pH-sensitive polymer.

3. Wound dressing according to Claim 2, **characterized in that** the particles have a diameter from 50 nm to 1000 pm, preferably 5 µm to 300 µm.

4. Wound dressing according to either of Claims 2 or 3, **characterized in that** the particles have a core that contains the Bronsted acid and that is encased in the pH-sensitive polymer.

5. Wound dressing according to either of Claims 2 or 3, **characterized in that** the particles comprise a matrix composed of the pH-sensitive polymer, in which the Bronsted acid is embedded.

6. Wound dressing according to any of the preceding claims, **characterized in that** the wound dressing additionally contains a substance that is a Bronsted base corresponding to said Bronsted acid and that is embedded together with the Bronsted acid in a matrix composed of the pH-sensitive polymer or is encased in the pH-sensitive polymer.

7. Wound dressing according to any of the preceding claims, **characterized in that** it includes a wound contact layer or consists of a wound contact layer.

8. Wound dressing according to any of the preceding claims, **characterized in that** it includes an absorbent layer or consists of an absorbent layer.

9. Wound dressing according to Claim 8, **characterized in that** the Bronsted acid is contained in the absorbent layer.

10. Wound dressing according to any of Claims 7 to 9, **characterized in that** the Bronsted acid is contained in the wound contact layer.

11. Wound dressing according to any of Claims 7 to 10, **characterized in that** the Bronsted acid is contained in an amount of 0.01-100 mmol per gram in each layer that contains the Bronsted acid.

## Revendications

1. Pansement contenant un acide de Brönsted et un polymère sensible au pH, **caractérisé en ce que**
l'acide de Brönsted est incorporé dans une matrice en le polymère sensible au pH, ou est entouré du polymère sensible au pH,
l'acide de Brönsted étant une substance choisie dans le groupe acide acétique, acide citrique, acide lactique, acide glycérique, acide gluconique, acide benzoïque, acide aconitique, acide glutarique, acide tartrique, acide phosphorique, acide malique, acide subérique et acide glutamique et
le polymère sensible au pH étant un polymère acide méthacrylique-co-méthacrylate de méthyle.

2. Pansement selon la revendication 1, **caractérisé en ce que** l'acide de Brönsted est contenu dans des particules qui comprennent l'acide de Brönsted et le polymère sensible au pH.

3. Pansement selon la revendication 2, **caractérisé en ce que** les particules ont un diamètre de 50 nm à 1 000 pm, de préférence de 5 µm à 300 µm.

4. Pansement selon l'une des revendications 2 ou 3, **caractérisé en ce que** les particules comprennent un noyau qui contient l'acide de Brönsted et est entouré du polymère sensible au pH.

5. Pansement selon l'une des revendications 2 ou 3, **caractérisé en ce que** les particules comprennent une matrice en le polymère sensible au pH, dans laquelle est incorporé l'acide de Brönsted.

6. Pansement selon l'une des revendications précédentes, **caractérisé en ce que** le pansement contient en outre une substance qui représente une base de Brönsted correspondant audit acide de Brönsted, et qui, conjointement à l'acide de Brönsted, est incorporée dans une matrice en le polymère sensible au pH ou est entourée du polymère sensible au pH.

7. Pansement selon l'une des revendications précédentes, **caractérisé en ce qu'**il contient une couche de contact avec la plaie ou est constitué d'une couche de contact avec la plaie.

8. Pansement selon l'une des revendications précédentes, **caractérisé en ce qu'**il contient une couche absorbante ou est constitué d'une couche absorbante.

9. Pansement selon la revendication 8, **caractérisé en ce que** l'acide de Brönsted est contenu dans la couche absorbante.

10. Pansement selon l'une des revendications 7 à 9, **caractérisé en ce que** l'acide de Brönsted est contenu dans la couche de contact avec la plaie.

11. Pansement selon l'une des revendications 7 à 10, **caractérisé en ce que** l'acide de Brönsted est contenu dans chaque couche contenant l'acide de Brönsted en une quantité de 0,01 à 100 mmol par gramme.
